(19) Europäisches Patentamt
European Patent Office
Office européen des brevets

(11) Publication number: **0 040 466**
**B1**

# (12) EUROPEAN PATENT SPECIFICATION

(45) Date of publication of patent specification: 11.11.87

(51) Int. Cl.⁴: **C 12 N 15/00,** C 07 H 21/04

(21) Application number: 81301304.2

(22) Date of filing: 26.03.81

## (54) Adaptor molecules for DNA and their application to synthesis of gene-derived products.

(30) Priority: 27.03.80 US 129880

(43) Date of publication of application:
25.11.81 Bulletin 81/47

(45) Publication of the grant of the patent:
11.11.87 Bulletin 87/46

(84) Designated Contracting States:
BE CH DE FR GB IT LI NL SE

(56) References cited:
EP-A-0 036 776
FR-A-2 392 033
GB-A-2 007 676
GB-A-2 073 245

NUCLEIC ACIDS RESEARCH - SYMPOSIUM SERIES, no. 7, Proceedings of the International Symposium on Chemical Synthesis of Nucleic Acids, held in Egestorf, GFR on 5th-8th May 1980, edited by H. Köster, IRL Press, August 1980, LONDON (GB). S. A. NARANG et al.:

(73) Proprietor: **Canadian Patents and Development Limited**
**275 Slater Street**
**Ottawa Ontario, K1A OR3 (CA)**

(72) Inventor: **Narang, Saran A.**
**30 Higgins Road**
**Ottawa, ON K2G OR5 (CA)**
Inventor: **Wu, Ray J.**
**111 Christopher Circle**
**Ithaca, NY 14850 (US)**

(74) Representative: **Lambert, Hugh Richmond et al**
**D. YOUNG & CO. 10 Staple Inn**
**London, WC1V 7RD (GB)**

**EP 0 040 466 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
"Synthesis of the human insulin gene. Part IV. New Synthetic deoxyribooligonucleotide adaptors and primer for DNA cloning and sequence analysis", pages 377-385

NUCLEIC ACIDS RESEARCH, vol. 5, no. 12, December 1978, IRL LONDON (GB). P. CHARNAY et al.: Bacteriophage lambda and plasmid vectors, allowing fusion of genes in each of the three translational phases", pages 4479-4494

BIOCHEMICAL AND BIOPHYSICAL RESEARCH COMMUNICATIONS, vol. 81, no. 3, April 14, 1978, Academic Press. C. P. BAHL et al.:

"Chemical synthesis of versatile adaptors for molecular cloning", pages 695-703
METHODS IN ENZYMOLOGY, vol. 68, 1979, Academic Press. R. J. Rothstein et al.:

"Synthetic adaptors for cloning DNA", pages 98-109

RECUEIL, JOURNAL OF THE ROYAL NETHERLANDS CHEMICAL SOCIETY, 98/11, November 1979. J. F. M. DE ROOIJ et al.: "Synthesis of complementary DNA fragments via phosphotriester intermediates", pages 537-548

NATURE (1979) vol.281, pp.544-548

## Description

Field of the Invention

This invention is concerned with DNA information sequences, novel adaptor molecules for these sequences and the joining of the resulting adapted sequences into replicable cloning vehicles. Of particular concern are genes and adaptd genes coding for the A and B chains of insulin, the insertion of these adapted genes into cloning vehicles, and the transferring of the hybrid DNA molecules into host cells, the transformed cells thus having the ability to produce the specified insulin chains.

Background and Prior Art

In very recent years, methods have been developed (see "Molecular Cloning of Recombinant DNA", eds., W. A. Scott and R. Werner, Academic Press Inc., 1977)

(1) for the *in vitro* joining by DNA ligase of a DNA segment to be cloned (scheme 1, structure I) to a cloning vehicle (DNA capable of independent replication, structure II),

(2) for introducing the hybrid DNA molecule (recombinant DNA, structure III) into a suitable cell,

(3) for selecting and identifying the transformed cells carrying the desired hybrid DNA (cloned DNA as a hybrid DNA, structure IV),

(4) for amplifying the desired cloned DNA in the transformed cells, and

(5) for expressing the cloned DNA as a protein product. In most reported cases, DNA molecules isolated from cells or viruses have been fragmented by restriction enzyme digestion or by physical shearing before cloning.

Scheme 1. The cloning of a DNA fragment in a bacterium.

The dots at the ends of DNA molecules represent the proper sequence that allows matching and ligation of the ends with other DNA molecules.

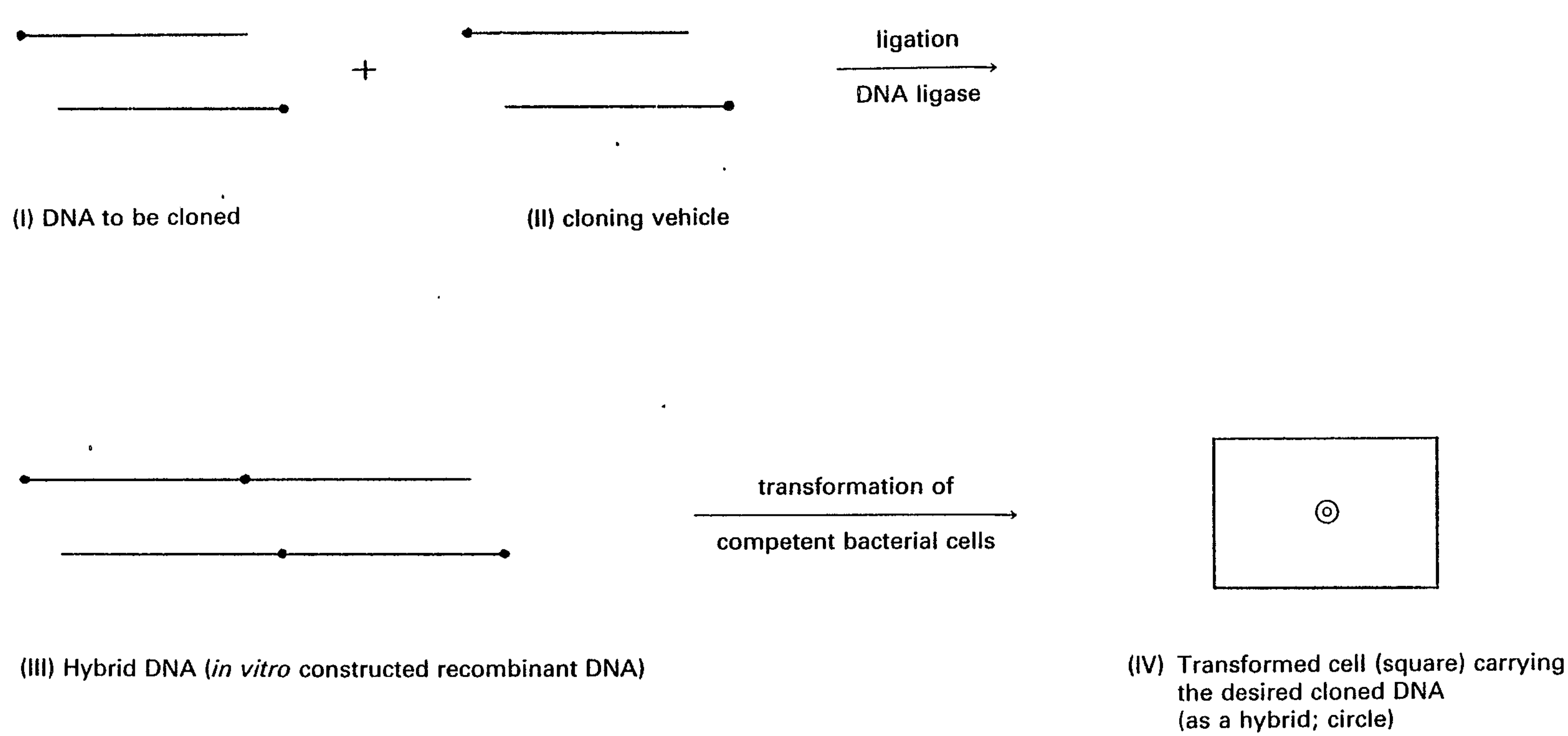

Protein synthesis in bacteria at a site located within a transferred DNA segment derived from mouse was shown by Chang et al (Cell *6,* 231—244, 1975). Still other examples of the cloning of natural foreing DNA have been described recently.

Methods for the total chemical synthesis of oligodeoxynucleotides of up to 20 nucleotides long, have been well established by using either the phosphodiester method (Khorana, H. G., J. Mol. Biol. *72,* 209, 1972) or the improved phosphotriester method (Hsiung, H. M., and Narang, S. A., Nucleic Acids Res. *6,* 1371, 1979; Narang, S. A. et al, Methods in Enzymology, Vol. 65, 610, 1979, and Vol. 68, 90, 1979). The latter method is now the preferred method because of its higher speed, better yield and purity of products, and has been used to prepare defined DNA sequences of longer length.

A few chemically-synthesised DNA sequences, such as the lactose operator (Marians, Wu et al, Nature 263, 744, 1976) and the tyrosine tRNA gene (Khorana, Science 203, 614, 1979), have been successfully cloned in *E coli* and the expression of the cloned DNA detected in subsequent cultures. Recent reports have indicated that human brain hormone somatostatin (14 amino acids) has been produced in a transformed bacterial host which had the transferred synthesized gene (Itakura et al, Science *198,* 1056, 1977). More recently, reports have appeared that human growth hormone has been produced in bacteria transformed with transferred genetic material comprising the appropriate gene. This latter gene was prepared by copying one DNA part from isolated mRNA (from human pituitary) and synthesizing the remaining part, then joining the two (Goedell et al, Nature *281,* 544, 1979).

In the pancreas of animals, preproinsulin (Chan, S. J. and Steiner, D. F., Proc. Nat. Acad. Sci. *73,* 1964, 1976) is synthesized as the precursor of insulin. The general structure of proinsulin (Formula 1) is $NH_2$—B chain-(C peptide)-A chain-COOH; it is converted to insulin by the action of peptidases in the pancreatic islet tissue which removed the C peptide by cleavage at the positions of the two arrows shown in Formula 1. The B-chain and A-chain of insulin are held together by two disulfide cross-linkages.

Using a biological method, Ullrich et al, (Science *196,* 1313, 1977) and Villa-Komoroff, et al, (Proc. Nat. Acad. Sci. *75,* 3727, 1978) succeeded in cloning the region of rat proinsulin I. However, the cloned gene included extraneous sequences and production of biologically active insulin was not achieved. Using a chemical method, Crea et al (Proc. Nat. Acad. Sci. *75,* 5765, 1978) synthesized, and Goeddel et al (Proc. Nat. Acad. Sci. *76,* 106, 1979) cloned, an insulin A-chain gene and a B-chain gene. The codons selected for these synthetic genes were arbitrary and quite different from the natural human DNA sequence. The construction of these genes was tedious. On culturing, the bacteria produced an insulin A-chain protein and B-chain protein which were separately treated to remove the extraneous β-galactosidase amd methionine. The production of A-chain and B-chain proteins was rather poor.

Formula 1. The structure of human proinsulin. B chain, amino acids 1-30; C peptide, amino acids 31-65; A chain, amino acids 66-86.

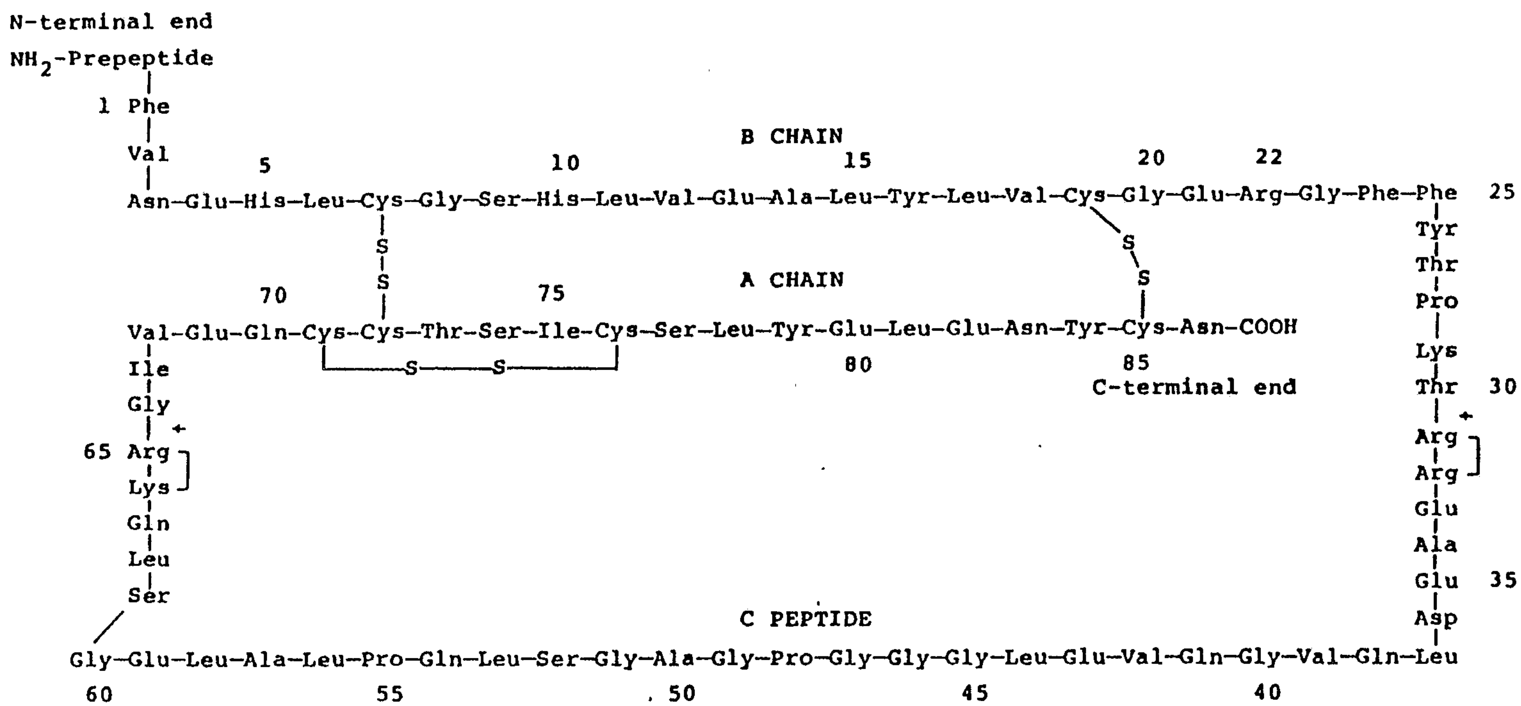

In addition to the foregoing, various authors have disclosed various methods of adapting DNA sequences for insertion into a cloning vehicle, by attaching "adaptor" molecules to the ends of the sequence to be inserted, such adaptors usually consisting of a chemically synthesised short DNA fragment which is added to the sequence to be inserted for a specific purpose.

For example, Goeddel et al in Nature Vol. 281 (1979), 544—548, describe at page 544 left-hand column, a chemically synthesised DNA adaptor fragment containing an ATG initiation codon and the coding sequences for residues 1—23 of the human growth hormone (HGH). This chemically synthesised adaptor was eventually combined with a second DNA fragment containing the coding sequences for residues 24—191 of HGH and obtained by HaeIII digestion of double-stranded HGHcDNA. These two fragments are first separately cloned and then combined to form a synthetic-natural hybrid gene, which when transformed into E. Coli. results in the expression of a polypeptide having similar characteristics to mature HGH.

In Methods in Enzymology, Vol. 68 (1979), 98—109, Rothstein et al disclose DNA adaptors of three main types: those containing a restriction enzyme recognition site, ready-made adaptors with one blunt end and one cohesive end, and conversion adaptors with two cohesive ends. The restriction recognition site adaptors are short chain, blunt ended duplexes which can be blunt end ligated onto a DNA sequence, thereby to introduce a restriction recognition site which can then be cleaved with the appropriate restriction enzyme to leave the DNA sequence with cohesive ends thereby facilitating its insertion into a replicable cloning vehicle.

In GB—A—2,007,676 human insulin A-chain and B-chain genes are disclosed containing start (ATG) and stop codons (TAA; TAG) at opposite ends of the respective DNA information sequence and downstream and upstream respectively of the restriction enzyme recognition sites producing cohesive sticky ends on the information sequence. Such stop and start codons and restriction sites are, however, incorporated as a result of the specific oligonucleotide blocks used to build up the gene, rather than as adaptors which are subsequently attached to the ends of a preformed, or preobtained gene sequence.

Finally, in both EP—A—0 036 776 and GB—A—2,073,245 symmetrical, palindromic DNA linker fragments are disclosed, viz. the fragment

CATGAATTCATG
GTACTTAAGTAC

which comprises duplicate start codons

CAT
GTA

on either side of an EcoRI site

GAATTC
CTTAAG

and which are used as a means of introducing an EcoRI site onto the ends of a DNA restriction fragment, to provide that fragment, after EcoRI digestion, with EcoRI cohesive ends.

In accordance with the present invention an improved adaptor is provided which enables stop and start codons to be attached to a gene sequence with improved efficiency. In particular the invention provides an adaptor molecule containing duplicate stop or start signals and which is palindromic so that it does not matter which way round it is ligated to the DNA sequence, and which, in the case of an adaptor comprising a start signal, contains one or more additional base pairs are inserted into the adaptor so that a correct leading frame is ensured in the adapted gene.

Broadly speaking, in accordance with the present invention adaptor oligodeoxynucleotide molecules are provided for facilitating insertion of DNA information sequences into a cloning vehicle and transcription and translation of said sequences in a host cell; each adaptor comprising a recognition site for a restriction endonuclease, and being characterised in that the molecule is a symmetrical DNA duplex molecule having either a start signal or a stop signal at each end, but not both, and a central recognition site between said start or stop signals, each complementary nucleotide strand having complete palindromic symmetry to its opposite strand, with both halves of the duplex being identical on 180° rotation of one half.

In each "start" adaptor, the start signal(s) will always be located down stream of the restriction endonuclease recognition site, in each "stop" adaptor, the stop signal(s) will always be located upstream of the recognition site. Each "start" or "stop" signal is one codon (base triplet) long, but more than one codon may be used together to reinforce the signal. The "start" or "stop" codons are selected and positioned to cause the desired DNA information sequence to be read or translated correctly and exclusively in every case thus to assure a high yield of mRNA and ultimate protein in the host cell. In the case of a start signal adaptor, the molecule will include at lest one additional base between each start signal and the recognition site to correct the frame shift of a DNA information sequence with inappropriate reading frame. The ATG codon is the basic "start" signal, although the GTG codon may be used instead.

The "stop" signal is one (or more) codon(s) selected to ensure that the translation in the host cell ceases at precisely the right location to give the correct protein (without subsequent modifications being required). Codons found to be particularly suitable are at least one from the group TGA, TAA and TAG.

The adaptors are preferably formed and used as separate molecules (Bahl et al, Gene *1*, 81, 1976), but in some cases, may be built up on the end of a DNA information sequence (Marians, Wu et al, Nature *263*, 744, 1976).

The adaptor molecules of this invention are short duplex DNA sequences including the recognition site and signal, the signal being at both ends of the duplex so that the complete adaptor molecules have rotational symmetry along a two-fold axis (X and Y). In other words, these symmetrical adaptors are DNA duplexes having duplicate signals at each end, each complementary nucleotide strand having complete palindromic symmetry to its opposite strand, with both halves of the duplex being identical on 180° rotation of one half. Either end of the adaptor can attach to the DNA with increased efficiency (see example with insulin A-chain below). Such start signal adaptors can include additional nucleotide bases between each start signal and the central recognition site, to correct the frame shift of a DNA information sequence with inappropriate reading frame.

Synthetic genes coding for the A-chain and B-chain of human-type insulin have been prepared, provided with selected "start" and "stop" adaptors as described above, and inserted into a replicable cloning vehicle. This vehicle or vector is extrachromosonal DNA which can enter a cell and replicate itself, such as a plasmid. The synthetic gene+cloning vehicle hybrid has been inserted into a host cell, and the transformed host cell progeny was shown to contain the exact input insulin A-chain or B-chain gene by DNA sequence analysis.

Also included within the scope of the present invention are a method of adapting a duplex DNA information sequence to facilitate the insertion thereof into a cloning vehicle, which comprises ligating an adaptor molecule to the duplex DNA information sequence, wherein the adaptor is a palindromic adaptor as above defined, and a method of inserting a duplex DNA information sequence into a replicable cloning vehicle, which comprises enzymatically ligating a start signal containing adaptor molecule to the upstream end of the information sequence which is to be inserted into the cloning vehicle, and a stop signal containing adaptor molecule to the downstream end, said adaptor molecules both containing a restriction enzyme recognition site, enzymatically digesting the adaptor molecules, after ligation to the said DNA information sequence, with the appropriate restriction enzyme to produce an adapted DNA sequence having cohesive ends, and enzymatically ligating the adapted DNA via its cohesive ends into the cloning vehicle.

Detailed Description and Preferred Embodiments

Typical adaptors having stop signals according to the invention can be depicted respectively as follows (in duplex form):

<u>Formula 2b</u>

| | <u>stop</u> | | |
|---|---|---|---|
| 5' | TGA | | TCA |
| | | recognition site | |
| 3' | ACT | | AGT |

<u>Formula 2c</u>

| | <u>stop</u> | | |
|---|---|---|---|
| 5' | TAA | | TTA |
| | | recognition site | |
| 3' | ATT | | AAT |

<u>Formula 2d</u>

| | <u>stop</u> | | |
|---|---|---|---|
| 5' | TAG | recognition site | CTA |
| 3' | ATC | | GAT |

These duplexes are seen to have rotational symmetry so that either end can attched to the genetic information sequence without side product formation. The recognition sites themselves have this rotational symmetry. A typical symmetrical duplex adaptor having a double stop signal and a Bam HI site is shown in Formula 2e:

Formula 2e

5′ TGA TAG GGATCC CTA TCA

3′ ACT ATC CCTAGG GAT AGTBam HI
site

The symmetrical adaptors can have an even number of from about 8 to about 20 or more nucleotide base pairs (or incomplete pairs) in their structure.

The enzyme recognition sites can be selected from those known for suitable restriction endonucleases (over 100 are now known). In the examples below, we have used as the recognition site (the boxed-in region) the

Bam HI site GGATCC
CCTAGG
and the Eco RI site GAATTC
CTTAAG.

The part of this sequence which serves as the protruding cohesive end after cleavage, is 5′ GATC for Bam HI, and 5′ AATT for Eco RI (the complete recognition sequence less the two end bases). The cohesive end portions of the recognition sequences of other restriction endonucleases are derived similarly depending on the digestion cleavage location. For example, for Hind III the protruding cohesive sequence would be AGCT; for Xba I, CTAG; and for Ava II, GAC or GTC. Other restriction endonucleases and their recognition sequences are described in, for example, the article by H. O. Smith in Science, Vol. 205, p. 455, 3 August 1979 (see Table 1, p. 458), and by R. J. Roberts, in Gene, Vol. 4, p. 183, 1978. Preferably the enzyme recognition sequence duplex has a cleavage pattern that, on digestion, leaves a protruding part of at least 3 bases, and more preferably 4. When used herein, "recognition site" is meant to include the partial site which is recognized and adhered to by the complementary portion.

The DNA information sequence may be a duplex DNA which can be isolated from a natural source (such as from microorganisms, plant cells or animal cells) it or may be synthesized chemically or enzymatically. It will code for some desired gene-derived product.

In some cases, the genetic information sequence to be transferred may have a faulty frame shift. By attaching one or more base pairs between the "start" signal and the enzyme recognition sequence on the adaptor molecules, the reading frame of the triplet code can be corrected by means of the adaptors. For example, the reading frame of the non-overlapping triplet genetic code as it follows through the cloning vehicle and adaptor sequence may end up at the start signal, e.g. as in Formula 3a.

Formula 3a

start

| | | | | |
|---|---|---|---|---|
| 5′ | ... | ATT | C¦AT G | DNA duplex |
| 3′ | ... | TAA | G¦TA C | |

This will cause the DNA information duplex to be read incorrectly. By inserting one or two nucleotide pairs (X, Y) in the adaptor (between the start signal and the recognition site) the reading frame can be shifted as shown in Formula 3B;

Formula 3b

start

| | | | | |
|---|---|---|---|---|
| ... | ATT C | ¦ XX | ATG | DNA duplex |
| ... | TAA G | ¦ YY | TAC | |

In some cases it may be desirable to insert one complete codon plus one or two nucleotide pairs (i.e. 4 or 5 nucleotide pairs). The X and Y can be any complementary nucleotide pair: with bacterial host cells T and A usually are preferred. These inserts will enable the DNA to be read correctly. In order to maintain a rotationally symmetrical adaptor corresponding base pairs are inserted on both sides of the enzyme recognition site. The advantage of such symmetrical adaptors is to assure the correct joining to DNA in every case and thus give good yields of the desired adapted DNA sequence (where full duplex is attached to DNA duplex). An example of such a symmetrical start signal adaptor is given in the following formula 3c:

Formula 3c

```
         Eco RI site     start
        ________________________
5'  CATAAGAATTCTTATG

3'  GTATTCTTAAGAATAC
    start
```

These adaptor sequences have been prepared by an improved phosphotriester method including techniques as described in Nucleic Acid Res. *7,* 2199, 1979, and in the Hsiung et al and Narang et al references mentioned above. An outline of the synthesis of a 12-nucleotide-long start signal adaptor is given below for formula 5a and for its digested form (left side of formula 3d). The duplex adaptors can be prepared, then joined to each end of the gene (or DNA duplex to be cloned) and digested to give the protruding cohesive ends. In some cases (particularly where the gene is being synthesized), steps can be designed to yield the protruding 5′ recognition site and signal without digesting the full adaptor duplex, as illustrated below for the insulin B-chain.

When attaching the first adaptor duplex to the gene duplex (as by DNA ligase), it has been found desirable, in order to minimize side products, to make the 5′-terminus at the opposite end of the gene 5′-OH. After joining this first adaptor, the 5′-OH end is made 5′-OP by using ATP and polynucleotide kinase, and the second adaptor joined. When the two adaptor duplexes are joined, the product is digested with the appropriate restriction enzyme(s) to produce the cohesive ends. The resulting digested adapted DNA sequence or gene will have the structure 3d (for example, where the Eco RI and Bam HI enzyme sites were used with the start and stop adaptors respectively). X and Y are the additional nucleotide inserts to correct the reading frame and Z is 1 or 2 (or 4 or 5).

Formula 3d

```
       RI site        start                   stop
5'     A A T T C  [X]  A T G  | DNA duplex |  T G A G            3'
3'             G  [Y]  T A C  |  sequence  |  A C T C C T A G    5'
                   Z                                 ____________
                                                     BamHI site
```

The adapted genetic information sequence having protruding cohesive ends which are part of the recognition site (and are recognized by the complementary part of the site), is now ready for insertion into a replicable cloning vehicle having complementary sites for cohesion. Suitable cloning vehicles may be plasmids, plasmid fragments, viruses, viral DNA fragments, and other cellular DNA pieces (usually at least about 3000 nucleotides long). The adapted DNA molecule (gene) and the cloning vehicle which has been treated with appropriate endonuclease, can be joined by use of a polynucleotide ligase such as $T_4$ ligase. Preferably the cloning vehicle DNA will include a transcription promoter sequence such as the lac promoter or tryptophan promoter placed upstream of the inserted gene.

A-chain Insulin Gene

Of particular concern are genes coding for human insulin A- and B-chains and these will be described in detail as preferred embodiments of the invention. The amino acid sequence of human insulin A-chain is shown in formula 4a upper line, from amino acid number 66 to 86. This sequence is identical to that of rat insulin A-chain except that amino acid no. 69 is aspartic acid in rat insulin. Ullrich et al (cited above) described the codon sequence of mRNA (and DNA) for rat insulin A-chain. Drawing in part from this sequence and from other knowledge, and using instead the codon GAG for glutamic acid, a mRNA and partial duplex DNA sequence was put together as shown in formula 4a and 4b. This sequence we selected is much closer to the natural human DNA sequence than that selected by Crea et al, mentioned above. We synthesized the DNA H strand of 43 nucleotides and separetely the DNA L strand of 36 nucleotides (as in formula 4a) using the phosphotriester method as described previously. The two strands were partially complementary and formed the partial duplex as shown. To complete the synthesis of the 63-nucleotide-long duplex DNA, we made use of primer-extension repair-synthesis techniques to complete the full duplex DNA gene. These repair-synthesis techniques are described by R. Wu et al, in Methods in Cancer Research *12,* 87, 1976. The overlapping of 16 nucleotides between the H- and L-strands was sufficient to allow efficient repair-synthesis by AMV reverse transcriptase at 37°C, or by *E. coli* DNA polymerase at 5—15°C. In this way, we reduced the rather time-consuming effort required for the chemical synthesis of the entire two strands. The sequence of the resulting DNA was analyzed in part and confirmed by the chemical method of Maxam and Gilbert (Proc. Nat. Acad. Sci. *74,* 560, 1977). This human A-chain gene differs from that described by Crea et al (cited above) by having some different codons which are closer to the natural codons used for human insulin gene and having no extragenous DNA sequences so that this "as synthesized" gene can be joined directly to insulin chain B—C gene to construct a proinsulin gene (chain B—C—A) with no improper codons intervening.

Formula 4a  Amino acid sequence of human insulin A chain and the corresponding mRNA and DNA sequence. The designation of the H- and L-strands of DNA are arbitrary.

```
                            66       70           76       80            86
Amino acids (A-chain)       Gly.Ile.Val.Glu.Gln.Cys.Cys.Thr.Ser.Ile.Cys.Ser.Leu.Tyr.Gln.Leu.Glu.Asn.Tyr.Cys.Asn
human insulin

mRNA                   5'   GGC.AUU.GUG.GAG.CAG.UGC.UGC.ACC.AGC.AUC.UGC.UCC.CUC.UAC.CAA.CUG.GAG.AAC.UAC.UGC.AAC   3'

DNA (H)                5'   GGC.ATT.GTG.GAG.CAG.TGC.TGC.ACC.AGC.ATC.TGC.TCC.CTC.TAC.C                             3'
                                5    10   15   20   25   30   35   40

DNA (L)                3'                                  TAG.ACG.AGG.GAG.ATG.GTT.GAC.CTC.TTG.ATG.ACG.TTG.   5'
                                                           36                                           1
```

Formula 4b  Construction of human insulin A-chain gene. The roman numerals indicate the specific oligodeoxynucleotide fragments synthesized chemically.

```
                                 I               II                III              VIII
DNA (H)                5'   *pGGC.ATT.GTG.GAG.CAG.TGC.TGC.ACC.AGC.ATC.TGC.TCC.CTC.TAC.C   G.GAG.AAC.TAC
                                 5    10   15   20   25   30   35   43
                                                           36                                         1
DNA (L)                3'             TC.GTC.ACG.ACG.T     TAG.ACG.AGG.GAG.ATG.GTT.GAC.CTC.TTG.ATG.ACG.TTG-OH   5'
                                          VII                   IV            V              VI
```

In constructing the synthetic human insulin A-chain gene, each of the chemically synthesized oligodeoxynucleotides (fragment I through VIII, formula 4b) was phosphorylated at its 5′-ends with $^{32}P$. The phosphorylation reaction for each oligodeoxynucleotide (2 nmoles) includes 50 mM glycine-NaOH (pH 9.5), 10 mM $MgCl_2$, 10 mM dithiothreitol, 5 nmoles of [$\gamma$-$^{32}P$]ATP of specific activity of around 20 mCi/µmole, and 3 units of $T_4$ polynucleotide kinase in a final volume of 50 µl. The reaction was carried out at 37°C for 2 hours; during this time from 50 to 70 percent of the oligodeoxynucleotide was phosphorylated. The desired product was purified by polyacrylamide gel electrophoresis (15% gel, under denaturing conditions).

The 43-nucleotide-long upper strand (see formula 4a, H strand) was assembled by annealing 500 pmoles each of 5′-phosphorylated oligonucleotide fragments I, II, III, IV and VII in 15 µl at 65° and slowly cooled to 0°C. Concentrated ligase buffer was added to give the following final concentrations: 50 mM Tris-HCl (pH 7.6), 10 mM $MgCl_2$, 10 mM dithiothreitol, 100 µM ATP. $T_4$ polynucleotide liagase (0.4 unit) was added and the mixture incubated at 12°C for 24 hours. The ligation product was purified on a 15% polyacrylamide gel (denaturing condition). The desired 43-long oligodeoxynucleotide migrated to about 46% that of the bromophenol blue dye marker. The yield of purified 43-long product after elution from the gel was around 50 pmoles. The 36-nucleotide-long lower strand (formula 4a, L strand) was assemble in a similar manner using phosphorylated oligonucleotide fragments III, IV, V, VIII and non-phosphorylated fragment VI.

The 63-nucleotide-long duplex (63-mer) A-chain insulin gene was constructed by annealing about 40 pmoles each of the 5′-$^{32}P$ 43-long and the 5′-OH 36-long oligodeoxynucleotides. Repair synthesis was carried out, to fill in the single-stranded regions, in 50 mM Tris-HCl (pH 8.3), 50 mH KCl, 10 mM $MgCl_2$, 10 mM dithiothreitol, 100 µg/ml bovine serum albumin, 100 µM each of dGTP, dATP, dTTP and [$\alpha$-$^{32}P$]dCTP (specific activity around 4 mCi/µmole), and 9 units of avian myeloblastosis virus reverse transcriptase in a final volume of 20 µl. Incubation was at 37°C for 3 hours. The desired products were purified by polyacrylamide gel electrophoresis and two strands of the 63-mer were separated (migrated to about 72% that of the xylene cyanol dye marker). DNA sequence analysis of the eluted 63-long products (pilot scale with high specific activity $^{32}$-P-dCTP) showed the correct sequence for the A-chain insulin gene.

The cloning of this insulin A-chain gene was controlled by adding both a start signal and a stop signal for protein synthesis (in the adaptors described above) to the two ends of the synthetic gene. Not only can this adapted DNA be joined to the cloning vehicle but after cloning and transcription, the initiation and termination of insulin protein (A-chain) synthesis can be achieved.

The dodecadeoxynucleotide adaptor duplex containing a start signal downstream of the restriction endonuclease recognition site was synthesized using the phosphotriester methods to have the structure:

Formula 5a

| | | Eco RI site | start |
|---|---|---|---|
| (H) | 5′ | C A T G A A T T C | A T G |
| (L) | 3′ | G T A C T T A A G | T A C |

Similarly, a stop signal adaptor was synthesized to have the structure:

Formula 5b

| | | stop | |
|---|---|---|---|
| (H) | 5′ | T G A | G G A T C C T C A |
| (L) | 3′ | A C T | C C T A G G A G T |
| | | | Bam HI site |

The 5′ terminus at the tail end of the A-chain gene was kept in its 5′-OH form and the start signal adaptor 5a joined to the head end using $T_4$ polynucleotide ligase by blunt-end ligation as follows. The reaction mixture contained 50 mM Tris-HCl :pH 7.6), 10 mM $MgCl_2$, 10 mM dithiothreitol, 100 µM ATP, 200 pmoles of the self-complementary 12-mer start-signal adaptor (5a), 25 pmoles of 63-mer duplex A-chain gene, and 0.25 units of $T_4$ polynucleotide kinase in a final volume of 15 µl. Incubation was at 12°C for 40 hours. Small scale gel electrophoresis of an aliquot of the reaction mixture with and without Eco RI enzyme digestion showed that about 50% of the 63-mer duplex was ligated to a start-signal adaptor. The DNA in the main portion of the reaction mixture was extracted with chloroform-isoamyl alcohol (24:1 v/v) and precipitated with 2 volumes of ethanol. The tail end 5′-OH was then converted to 5′-OP using $ATP^{32}$ and polynucleotide kinase (as before). Next the stop signal adaptor 5b was ligated to the tail end of the A-chain gene to give the adapted gene. The adapted gene was digested successively with Eco RI and Bam HI restriction enzymes to produce the respective cohesive ends (and to remove any additional start or stop signals ligated to the gene).

A cloning vehicle was prepared, by cutting out and purifying on an agarose gel, a 4000-nucleotide-long fragment from pBR 322 plasmid with both Eco RI and Bam HI enzymes. This fragment (0.5 μg) had cohesive ends which were then ligated by μ ligase (as before) to the complementary part of the enzyme recognition sites on the adapted insulin A-chain gene (about 1 pmole).

This gene-cloning vehicle hybrid [one-half of the sample) was used to transform cell of *E. coli* (strain 5436). This strain and the containment facilities used conformed to current recombinant DNA regulations. One-hundred and ten ampicillin (trademark)-resistant and tetracycline-sensitive transformants were isolated. On being hybridized with $^{32}$P-labelled short synthetic insulin A-chain DNA fragments (30-35 bases long) by colony hybridization (Grunstein & Hogness, Proc. Nat. Acad. Sci. *72*, 3961, 1975) 22 positive clones were obtained. Direct DNA sequence analysis (Maxam and Gilbrt method) was carried out on several of the positive clones to confirm the presence of the insulin A-chain gene in the hybrid plasmid DNA. The DNA fragment excised, by digestion with both Eco RI and Bam HI enzymes, from three clones showed by direct DNA sequence analysis the exact nucleotide sequence as synthesized.

The cloned insulin A-chain gene on pBR 322 plasmid was prepared in increased quantities by growing *E. coli* cells carrying this hybrid plasmid in M9 medium until the optical density of the cell culture equalled about 1 at 600 nm. Chloramphenicol was added to give a final concentration of 150 mg/L. The cells were shaken at 37°C for another 12 to 16 hours. The hybrid plasmid DNA in the cells was isolated according to standard procedure (such as that of Marians et al, Nature *263*, 744, 1976).

The cloned insulin A-chain gene was next excised by digestion of the cloned hybrid plasmid DNA with Eco RI and Bam HI endonucleases. The Bam HI end was converted to an Eco RI and by using an Eco RI-Bam HI conversion adaptor of the type reported by Bahl et al (Biochem. Biophys. Res. Commun. *81*, 695, 1978) except that the double-stranded portion of the adaptor was increased from 6-long to 8- or 10-long to increase the efficiency of ligation. The conversion adaptor has the structure:

Formula 5c

```
     BamHI
     site
5'   GATC CGA CTG TTA G

3'         GCT GAC AAT C TTAA
                         EcoRI site
```

The resulting adapted insulin A-chain gene carrying an Eco RI site at each end, with the following type of structure:

Formula 5d

```
      EcoRI
      site   start                  stop
5'    AATTC  ATG  | DNA duplex |    TGA G GATC CGA CTG TTA G        3'
3'           G TAC|  sequence  |    ACT C CTAG GCT GAC AAT CTTA     5'
                                          BamHI            EcoRI
                                          site             site
```

can be ligated to a plasmid such as pBGP120 (Polisky et al, Proc. Nat. Acad. Sci. *73*, 3900, 1976), or a high copy number plasmid (such as pKN402 of Uhlin et al, Gene *6*, 91, 1969) carrying a strong promoter (such as the lactose promoter, the strain being constructed in our laboratory) for expression. This reconstructed hybrid plasmid DNA can be ligated at the Eco RI sistes and used to transform *E. coli* cells to direct the synthesis of large amounts of the human insulin A-chain protein. The flow of information from DNA to protein is by transcribing the DNA sequence into a complementary messenger RNA (mRNA sequence) and then translating the mRNA sequence (using the 3 letter genetic code) in the synthesis of a protein.

Still other plasmids or replicable DNA sequences would be operative as cloning vehicles.

For good yields of such transferred-gene-derived products, the culture medium for the host cells should be one that favours the reproduction of the inserted DNA hybrid, as well as transcription, translation and expression of desired product. Such culture media can be of the type designated M9 as described in J. Bacteriol. *119*, 450, 1973, L. Katz and D. R. Helinski.

These media contain nutrients that favour the replication of plasmids and other replicable cloning vehicles (as well as protein synthesis). Further such media are described in "Experiments in Molecular Genetics" by J. M. Miller, page 431, 1972, Cold Spring Harbour Lab., N.Y.

B-Chain Insulin Gene

The chemical synthesis of a gene coding for the B-chain of human insulin is outlined in formula 6, the Roman numerals indicating the portions as synthesized by the phosphotriester method (H. M. Hsiung, S. A. Narang et al, Nucleic Acids Res. *6*, 1371, 1979) and their sequence of joining by DNA ligase. The portions

can be joined by DNA ligase, and the DNA partial duplex completed by repair synthesis to form the full duplex, in a manner similar to the assembly of the insulin A-chain fragments. Both a start signal and a stop signal adaptor can be added to the two ends of the synthetic gene as in the construction of the insulin A-chain gene. The synthesized B-chain gene, with protruding cohesive ends as in formula 6, can be ligated to the samed 4000-nucleotide-long duplex plasmid DNA as for the A-chain and the hybride used to transform *E. coli* strain 5346 cells as before.

An adapted B-chain gene based on the structure as in formula 6, was constructd by preparing

Formula 6

Sequence of fragments I to VII and X to XVI which were completed to constitute human-like insulin B-chain DNA coding sequence

```
        ——— I ————————————— II ————————————————— III ——————————————
5'  TTT GTC  AAT  CAG | CAC  CTT  TGT  GGT | TCT  CAC  CTG  GTG | GAG
3'               TA   GTC  GTG  GAA | ACA  CCA  AGA  GTG | GAC  CAC  CTC
             ——————— X ——————————————— XI ——————————————— XII ———

     —— IV ——————————————— V ——————————————— VI ——————————————— VII
5'  GCT  CTG  TAC | CTG  GTG  TGT  GGG  GAA  CGT  GGT  TTC  TTC  TAC
3'  CGA | GAC  ATG  GAC  CAC | ACA  CCC  CTT  GCA | CCA  AAG  AAG  ATG
    ———————— XIII ——————————— XIV ——————————— XV ————————

5'  ACA  CC
3'  TGT  GGG  TTC  TGG
    ———— XVI ————
```

segments and joining in sequence as shown by roman numerals, ligated to the plasmid, and transferred into host cells as described above. Transformed cells and the presence of the B-chain gene therein, were identified and confirmed as before.

Purified insulin A-chain protein, isolated from *E. coli* carrying the A-chain gene, can be converted to the S-sulfonate form and combined *in vitro* with the SH-form of similarly-isolated B-chain protein, or conversely, the B-chain in the S-sulfonate form can be combined *in vitro* with the SH-form of similarly isolated A-chain protein, according to the method of Katsoyannis et al, in J. Am. Chem. Soc. *95*, 6427, 1973, to form human-type insulin.

The host cells (any cell that allows the DNA to enter, to replicate and to produce gene-directed product) into which the adapted gene-cloning vehicle hybrid is transferred can be chosen from among various other bacteria, such as *Bacillus subtilis*; various fungi, e.g. yeasts, particularly baker's or brewer's yeasts; or plant or animal cells.

The transformed *E. coli* cells carrying the insulin A-chain and B-chain gene are maintained at the Biochemistry Department, Cornell University, Ithaca, N.Y., and are available upon request subject only to present or future Federal Government regulations on the handling of such materials.

The transformed *E. coli* cells carrying the insulin A-chain and B-chain genes have also been deposited with the American Type Culture Collection at ATCC nos. 31850 and 31851 respectively.

**Claims**

1. An adaptor oligodeoxynucleotide molecule, for facilitating insertion of DNA information sequences into a cloning vehicle, and transcription and translation of said sequences in a host cell, each adaptor molecule comprising a recognition site for a restriction endonuclease, characterised in that the molecule is a symmetrical DNA duplex molecule having either a start signal or a stop signal at each end, but not both, and a central recognition site between said start or stop signals, each complementary nucleotide strand having complete palindromic symmetry to its opposite strand, with both halves of the duplex being identical on 180° rotation of one half, with the proviso that when said signals are start signals at least one additional nucleotide base is included between each start signal and the central recognition site.

2. An adaptor molecule according to claim 1, comprising from 8 to 20 nucleotide base pairs.

3. An adaptor molecule according to claim 1 or 2, wherein the said recognition site is selective for one or other of the endonucleases Eco RI, Bam HI, and Hind III.

4. An adaptor molecule according to any one of claims 1-3 wherein said signals are start signals selected from an ATG codon and a GTG codon.

5. A start signal adaptor molecule according to claim 4, characterised by the structure:

```
     EcoRI site   start
     ------------------
5'   CATAGAATTCTATG

3'   GTATCTTAAGATAC
     start
```

6. A start signal adaptor molecule according to claim 4, characterised by the structure:

```
     EcoRI site     start
     --------------------
5'   CATAAGAATTCTTATG

3'   GTATTCTTAAGAATAC
     start
```

7. An adaptor molecule according to claim 1 or 2, which comprises duplicate stop signals, said stop signals comprising a TGA, TAA or TAG codon.

8. A stop signal adaptor molecule according to claim 7, characterised by the structure:

```
        stop
   5'   TGA       ___________      TCA
                 | recognition |
                 |    site     |
   3'   ACT       -----------      AGT
        stop
   5'   TAA       ___________      TTA
                 | recognition |
                 |    site     |
   3'   ATT       -----------      AAT
        stop
   5'   TAG       ___________      CTA
                 | recognition |
                 |    site     |
   3'   ATC       -----------      GAT
or
   5'   TGA TAG   ___________      CTA TCA
                 | recognition |
                 |    site     |
   3'   ACT ATC   -----------      GAT AGT
```

9. A stop signal adaptor molecule according to claim 7, characterised in that the recognition site has the following structure:

```
5'   GGATCC

3'   CCTAGG
     Bam HI site
```

10. A method of adapting a duplex DNA information sequence to facilitate the insertion thereof into a cloning vehicle, which comprises ligating an adaptor molecule to the duplex DNA information sequence, characterised in that the adaptor molecule ligated to the duplex DNA information sequence is an adaptor molecule as claimed in any one of claims 1-9.

11. A method of adapting a duplex DNA information sequence according to claim 10, which comprises ligating a start signal containing adaptor molecule to the upstream end of the duplex DNA information sequence, and a stop signal containing adaptor molecule to the downstream end, said stop signal adaptor, at least, being a stop signal adaptor according to claim 1.

12. A method according to claim 10, or 11, characterised in that the stop signal containing adaptor molecule is a molecule as claimed in claim 7, 8 or 9.

13. A method according to claim 10 or 11, characterised in that the start signal containing adaptor molecule is a molecule as claimed in claim 4, 5 or 6.

14. A method according to any one of claims 9-13, characterised in that said duplex DNA information sequence is a gene or a partial gene.

15. A method according to claim 14, characterised in that said sequence is a gene sequence coding for the insulin A-chain or the insulin B-chain.

16. A method according to claim 15, characterised in that said information sequence is an insulin A-chain coding sequence of the structure:

5'GGC.ATT.GTG.GAG.CAG.TGC.TGC.ACC.AGC.ATC.TGC.TCC.CTC.TAC.CAA.
3'CCG.TAA.CAC.CTC.GTC.ACG.ACG.TGG.TCG.TAG.ACG.AGG.GAG.ATC.GTT.

CTG.GAG.ACC.TAC.TGC.AAC.
GAC.CTC.TGG.ATG.ACG.TTG.

17. A method according to claim 15, characterised in that said information sequence is an insulin B-chain coding sequence of the structure:

5' TTT GTG AAT CAG CAC CTT TGT GGT TCT CAC CTG GTG GAG
3' AAA CAG TTA GTC GTG GAA ACA CCA AGA GTG GAC CAC CTC
5'-GCT CTG TAC CTG GTG TGT GGG GAA CGT GGT TTC TTC TAC
3'-CGA GAC ATG GAC CAC ACA CCC CCT GCA CCA AAG AAG ATG
5'-ACA CCC AAG ACC
3' TGT GGG TTG TGG

18. A method according to any one of claims 11-17, which includes the additional step of cleaving the adaptor molecules, after ligation onto the DNA information sequence, into partial duplex form at their respective restriction sites by the action of the appropriate restriction enzyme, thereby to provide a DNA information sequence with protruding cohesive ends derived from said adaptors.

19. A method of inserting a duplex DNA information sequence into a replicable cloning vehicle, which comprises enzymatically ligating a start signal containing adaptor molecule to the upstream end of the information sequence which is to be inserted into the cloning vehicle, and a stop signal containing adaptor molecule to the downstream end, said adaptor molecules both containing a restriction enzyme recognition site, enzymatically digesting the adaptor molecules, after ligation to the said DNA information sequence, with the appropriate restriction enzyme to produce an adapted DNA sequence having cohesive ends, and enzymatically ligating the adapted DNA via its cohesive ends into the cloning vehicle, characterised in that the adaptor molecules are molcules according to any one of claims 1-9.

20. A method according to claim 19, characterised in that the stop signal containing adaptor molecule is a molecule according to claim 7, 8 or 9, and the start signal containing adaptor molecule is a molecule according to claim 4, 5 or 6.

21. A method according to claim 19 or 20, characterised in that the DNA information sequence to be inserted is a gene or partial gene.

22. A method according to claim 21, characterised in that the DNA information sequence to be inserted is a gene sequence coding for the insulin A-chain or the insulin B-chain.

23. A method according to claim 22, characterised in that the DNA information sequence to be inserted is an insulin A-chain coding sequence of the structure set out in claim 16, or an insulin B-chain coding sequence of the structure set out in claim 17.

24. A method according to any one of claims 19—23, characterised in that the DNA information sequence is inserted into the replicable cloning vehicle downstream of a strong mRNA transcription promoter sequence.

**Patentansprüche:**

1. Adapter-Oligodeoxynukleotidmolekül zur Erleichterung der Einfügung von DNA-Informationssequenzen in einen Klonierungsvektor und zur Transkription und Translation dieser Sequenzen in einer Wirtszelle, wobei jedes Adaptermolekül eine Erkennungsselle für eine Hemm-Endonuklease aufweist, dadurch gekennzeichnet, daß das Molekül ein symmetrisches DNA-Doppelmolekül ist, das an jedem Ende Entweder ein Startsignal oder ein Stoppsignal, aber nicht beides, und eine mittige Erkennungsstelle zwischen diesen Start- oder Stoppsignalen hat, wobei jeder komplementäre Nukleotidstrang vollständige Palindromsymmetrie zu seinem Gegenstrang hat, wobei beide Hälften des Deoppelmoleküls bei Drehung der einen Hälfte um 180° identisch sind und wobei, wenn die genannten Signale Startsignale sind, wenigstens eine susätzliche Nukleotidbase zwischen jedem Startsignal und der mittigen Erkennungsstelle eingeschlossen ist.

2. Adaptermolekül nach Anspruch 1 mit 8 bis 20 Nukleotidbasenpaaren.

3. Adaptermolekül nach Anspruch 1 oder 2, bei dem die Erkennungsstelle für die eine oder andere der EndonukleasenEco RI, Bam HI, and Hind III selektiv ist.

4. Adaptermolekül nach einem der Ansprüche 1 bis 3, worin die Signale Startsignale sind, die unter einem ATG-Codon und einem GTG-Codon ausgewählt sind.

5. Startsignal-Adaptermolekül nach Anspruch 4, gekennzeichnet durch die Struktur

```
   EcoRI-Stelle  Start
5' CATAGAATTCTATG
3' GTATCTTAAGATAC
   Start
```

6. Startsignal-Adaptermolekül nach Anspruch 4, gekennzeichnet durch die Struktur

```
   EcoRI-Stelle  Start
5' CATAAGAATTCTTATG
3' GTATTCTTAAGAATAC
   Start
```

7. Adaptermolekül nach Anspruch 1 oder 2 mit doppelten Stoppsignalen, wobei diese Stoppsignale ein TGA-, TAA- oder TAG-Codon aufweisen.

8. Stoppsignal-Adaptermolekül nach Anspruch 7, gekennzeichnet durch die Struktur

```
        Stop
5'      TGA                        TCA
              [Erkennungs-
               stelle]
3'      ACT                        AGT

        Stop
5'      TAA                        TTA
              [Erkennungs-
               stelle]
3'      ATT                        AAT

        Stop
5'      TAG                        CTA
              [Erkennungs-
               stelle]
3'      ATC                        GAT
oder
5'      TGA TAG                    CTA TCA
              [Erkennungs-
               stelle]
3'      ACT ATC                    GAT AGT
```

9. Stoppsignal-Adaptermolekül nach Anspruch 7, dadurch gekennzeichnet, daß die Erkennungsstelle folgende Struktur hat

```
5' GGATCC
3' CCTAGG
   Bam HI-Stelle
```

10. Verfahren zur Anpassung einer Doppel-DNA-Informationssequenz, um ihre Einfügung in einen Klonierungsvektor zu erleichtern, indem man ein Adaptermolekül an die Doppel-DNA-Informationssequenz bindet, dadurch gekennzeichnet, daß das an die Doppel-DNA-Informationssequenz gebundene Adaptermolekül ein in einem der Ansprüche 1 bis 9 beanspruchtes Adaptermolekül ist.

11. Vefahren zur Anpassung einer Doppel-DNA-Informationssequenz nach Anspruch 10, bei dem man ein ein Startsignal enthaltendes Adaptermolekül an das Aufstromende einer Doppel-DNA-Informationssequenz und ein ein Stoppsignal enthaltendes Adaptermolekül an das Abstromende bindet, wobei wenigstens der Stoppsignaladapter ein Stoppsignaladapter gemäß Anspruch 1 ist.

12. Verfahren nach Anspruch 10 oder 11, dadurch gekennzeichnet, daß das ein Stoppsignal enthaltende Adaptermolekül ein in Anspruch 7, 8 oder 9 beanspruchtes Molekül ist.

13. Verfahren nach Anspruch 10 oder 11, dadurch gekennzeichnet, daß das ein Startsignal enthaltende Adaptermolekül ein in Anspruch 4, 5 oder 6 beanspruchtes Molekül ist.

14. Verfahren nach einem der Ansprüche 9 bis 13, dadurch gekennzeichnet, daß die Doppel-DNA-Informationssequenz ein Gen oder ein Teilgen ist.

15. Verfahren nach Anspruch 14, dadurch gekennzeichnet, daß die Sequenz eine Gensequenz ist, die die Insulin-A-Kette oder die Insulin-B-Kette codiert.

16. Verfahren nach Anspruch 15, dadurch gekennzeichnet, daß die Informationssequenz eine Insulin-A-Ketten-Codiersequenz der Struktur

5'GGC.ATT.GTG.GAG.CAG.TGC.TGC.ACC.AGC.ATC.TGC.TCC.CTC.TAC.CAA.
3'CCG.TAA.CAC.CTC.GTC.ACG.ACG.TGG.TCG.TAG.ACG.AGG.GAG.ATC.GTT.

CTG.GAG.ACC.TAC.TGC.AAC.
GAC.CTC.TGG.ATG.ACG.TTG. ist

17. Verfahren nach Anspruch 15, dadurch gekennzeichnet, daß die Informationssequenz eine Insulin-B-Ketten-Codiersequenz der Struktur

5' TTT GTG AAT CAG CAC CTT TGT GGT TCT CAC CTG GTG GAG
3' AAA CAG TTA GTC GTG GAA ACA CCA AGA GTG GAC CAC CTC
5'-GCT CTG TAC CTG GTG TGT GGG GAA CGT GGT TTC TTC TAC
3'-CGA GAC ATG GAC CAC ACA CCC CCT GCA CCA AAG AAG ATG
5'-ACA CCC AAG ACC
3' TGT GGG TTG TGG ist.

18. Verfahren nach einem der Ansprüche 11 bis 17 mit der zusätzlichen Stufe einer Spaltung der Adaptermoleküle nach Bindung an die DNA-Informationssequenz in eine Teildoppelform an ihren jeweiligen Kennstellen durch die Wirkung des entsprechenden Hemmenzyms, um so eine DNA-Informationssequenz mit von den Adaptern herrührenden vorspringenden zusammenhaltenden Enden zu bekommen.

19. Verfahren zur Einfügung einer Doppel-DNA-Informationssequenz in einen replizierbaren Klonierungsvektor, indem man enzymatisch ein ein Startsignal enthaltendes Adaptermolekül an das Aufstromende der Informationssequenz, die in den Klonierungsvektor eingefügt werden soll, und ein ein Stoppsignal enthaltendes Adaptermolekül an das Abstromende bindet, wobei die Adaptermoleküle beide eine Hemmenzym-Erkennungsstelle enthalten, enzymatisch die Adaptermoleküle nach Bindung an die DNA-Informationssequenz mit dem entsprechenden Hemmenzym zer legt, um eine angepaßte DNA-Sequenz mit zusammenhaltenden Enden zu erzeugen, und enzymatisch die angepaßte DNA über ihre zusammenhaltenden Enden in den Klonierungsvektor bindet, dadurch gekennzeichnet, daß die Adaptermoleküle Moleküle nach einem der Ansprüche 1 bis 9 sind.

20. Verfahren nach Anspruch 19, dadurch gekennzeichnet, daß das ein Stoppsignal enthaltende Adaptermolekül ein Molekül nach Anspruch 7, 8 oder 9 und das ein Startsignal enthaltende Adaptermolekül ein Molekül nach Anspruch 4, 5 oder 6 ist.

21. Verfahren nach Anspruch 19 oder 20, dadurch gekennzeichnet, daß die einzufügende DNA-Informationssequenz ein Gen oder Teilgen ist.

22. Verfahren nach Anspruch 21, dadurch gekennzeichnet, daß die einzufügende DNA-Informationssequenz eine Gensequenz ist, die die Insulin-A-Kette oder die Insulin-B-Kette codiert.

23. Verfahren nach Anspruch 22, dadurch gekennzeichnet, daß die einzufügende DNA-Informationssequenz eine Insulin--A-Ketten-Codiersequenz der in Anspruch 16 angebenen Struktur oder eine Insulin-B-Ketten-Codiersequenz der in Anspruch 17 angegebenen Struktur ist.

24. Verfahren nach einem der Ansprüche 19 bis 23, dadurch gekennzeichnet, daß die DNA-Informationssequenz in den replizierbaren Klonierungsvektor abstromwärts von einer starken mRNA-Transkription-Promotorsequenz eingefügt wird.

**Revendications**

1. Molécule adaptatrice oligodésoxynucléotidique, destinée à faciliter l'insertion de séquences d'information d'ADN dans un vecteur de clonage, et la transcription et la traduction desdites séquences dans une cellule hôte, chaque molécule adaptatrice comprenant un site de reconnaissance d'une endonucléase de restriction, caractérisée en ce que la molécule est une molécule duplex symétrique d'ADN ayant un signal de départ ou un signal d'arrêt, mais non les deux, à chaque extrémité et un site de reconnaissance central entre lesdits signaux de départ ou d'arrêt, chaque brin nucléotidique

complémentaire présentant une symétrie palindromique totale par rapport à son brin opposé, les deux moitiés du duplex étant identiques par rotation à 180° d'une moitié, sous réserve que, lorsque lesdits signaux sont des signaux de départ, au moins une base nucléotidique supplémentaire est incorporée entre chaque signal de départ et le site de reconnaissance central.

2. Molécule adaptatrice suivant la revendication 1, comprenant 8 à 20 paires de bases nucléotidiques.

3. Molécule adaptatrice suivant la revendication 1 ou 2, dans laquelle le site de reconnaissance est sélectif pour une endonucléase choisie entre EcoRI, BamHI et HindIII.

4. Molécule adaptatrice suivant l'une quelconque des revendications 1 à 3, dans laquelle les signaux sont des signaux de départ choisis entre un codon ATG et un codon GTG.

5. Molécule adaptatrice portant un signal de départ, suivant la revendication 4, caractérisée par la structure:

Site EcoRI Départ

5' CATAGAATTCTATG

3' GTATCTTAAGATAC
départ

6. Molécule adaptatrice portant un signal de départ, suivant la revendication 4, caractérisée par la structure:

Site EcoRI Départ

5' CATAAGAATTCTTATG

3' GTATTCTTAAGAATAC
départ

7. Molécule adaptatrice suivant la revendication 1 ou 2, qui comprend des signaux d'arrêt en double, signaux d'arrêt comprenant un codon TGA, TAA ou TAG.

8. Molécule adaptatrice portant un signal d'arrêt suivant la revendication 7, caractérisée par la structure:

| | | | |
|---|---|---|---|
| 5' | Arrêt<br>TGA | Site de reconnaissance | TCA |
| 3' | ACT | | AGT |
| 5' | Arrêt<br>TAA | Site de reconnaissance | TTA |
| 3' | ATT | | AAT |
| 5' | Arrêt<br>TAG | Site de reconnaissance | CTA |
| 3' | ATC | | GAT |
| 5' | TGA TAG | Site de reconnaissance | CTA TCA |
| 3' | ACT ATC | | GAT AGT |

9. Molécule adaptatrice portant un signal d'arrêt suivant la revendication 7, caractérisée en ce que le site de reconnaissance possède la structure suivante:

5' GGATCC

3' CCTAGG
Site Bam HI

10. Méthode d'adaptation d'une séquence d'information d'ADN duplex pour faciliter son insertion dans un vecteur de clonage, qui consiste à effectuer une ligation d'une molécule adaptatrice à la séquence d'information d'ADN duplex, caractérisée en ce que la molécule adaptatrice fixée par ligation à la séquence d'information d'ADN duplex est une molécule adaptatrice suivant l'une quelconque des revendications 1 à 9.

11. Méthode d'adaptation d'une séquence d'information d'ADN duplex suivant la revendication 10, qui consiste à effectuer une ligation d'une molécule adaptatrice contenant un signal de départ à l'extrémité amont de la séquence d'information d'ADN duplex, et une molécule adaptatrice contenant un signal d'arrêt à l'extrémité aval, ledit adaptateur portant un signal d'arrêt étant au moins un adaptateur portant un signal d'arrêt suivant la revendication 1.

12. Méthode suivant la revendication 10 ou 11, caractérisée en ce quel la molécule adaptatrice contenant un signal d'arrêt est une molécule suivant la revendication 7, 8 ou 9.

13. Méthode suivant la revendication 10 ou 11, caractérisée en ce que la molécule adaptatrice contenant un signal de départ est une molécule suivant la revendication 4, 5 ou 6.

14. Méthode suivant l'une quelconque des revendications 9 à 13, caractérisée en ce que la séquence d'information d'ADN duplex est un gène ou un gène partiel.

15. Méthode suivant la revendication 14, caractérisée en ce que la séquence est une séquence de gène codant pour la chaîne A de l'insuline ou la chaîne B de l'insuline.

16. Méthode suivant la revendication 15, caractérisée en ce que la séquence d'information est une séquence codant pour la chaîne A de l'insuline, de structure:

5'GGC.ATT.GTG.GAG.CAG.TGC.TGC.ACC.AGC.ATC.TGC.TCC.CTC.TAC.CAA.
3'CCG.TAA.CAC.CTC.GTC.ACG.ACG.TGG.TCG.TAG.ACG.AGG.GAG.ATC.GTT.

CTG.GAG.ACC.TAC.TGC.AAC.
GAC.CTC.TGG.ATG.ACG.TTG.

17. Méthode suivant la revendication 15, caractéisée en ce que la séquence d'information est une séquence codant pour la chaîne B de l'insuline, de structure:

5' TTT GTG AAT CAG CAC CTT TGT GGT TCT CAC CTG GTG GAG
3' AAA CAG TTA GTC GTG GAA ACA CCA AGA GTG GAC CAC CTC
5'-GCT CTG TAC CTG GTG TGT GGG GAA CGT GGT TTC TTC TAC
3'-CGA GAC ATG GAC CAC ACA CCC CCT GCA CCA AAG AAG ATG
5'-ACA CCC AAG ACC
3' TGT GGG TTG TGG

18. Méthode suivant l'une quelconque des revendications 11 à 17, qui comprend l'étape supplémentaire de clivage des molécules adaptatrices, après ligation sur la séquence d'information d'ADN, en une forme duplex partielle à leurs site de restriction respectifs par l'action de l'enzyme de restriction approprié, afin de produire de ce fait une séquence d'information d'ADN, ayant des extrémités cohésives en saillie, provenant desdits adaptateurs.

19. Méthode d'insertion d'une séquence d'information d'ADN duplex dans un vecteur de clonage réplicable, qui consiste à effectuer une ligation par voie enzymatique d'une molécule adaptatrice contenant un signal de départ à l'extrémité amont de la séquence d'information qui doit être insérée dans le vecteur de clonage, et une molécule adaptatrice contenant un signal d'arrêt à l'extrémité aval, les deux molécules adaptatrices contenant chacune un site de reconnaissance d'enzyme de restriction, à effectuer une digestion enzymatique des molécules adaptatrices, après ligation à ladite séquence d'information d'ADN, avec l'enzyme de restriction approprié pour produire une séquence d'ADN adaptée ayant des extrémités cohésives, et à effectuer une ligation par voie enzymatique de l'ADN adapté par ses extrémités cohésives dans le vecteur de clonage, méthode caractérisée en ce que les molécules adaptatrices sont des molécules suivant l'une quelconque des revendications 1 à 9.

20. Méthode suivant la revendication 19, caractérisée en ce que la molècule adaptatrice contenant le signal d'arrêt est une molécule suivant la revendication 7, 8 ou 9, et la molécule adaptatrice contenant le signal de départ est une molécule suivant la revendication 4, 5 ou 6.

21. Méthode suivant la revendication 19 ou 20, caractérisée en ce que la séquence d'information d'ADN à insérer est un gène ou un gène partiel.

22. Méthode suivant la revendication 21, caractérisée en ce que la séquence d'information d'ADN à insérer est une séquence de gène codant pour la chaîne A de l'insuline ou la chaîne B de l'insuline.

23. Méthode suivant la revendication 22, caractérisée en ce que la séquence d'information d'ADN à insérer est une séquence codant pour la chaîne A de l'insuline, ayant la structure indiquée dans la revendication 16, ou une séquence codant pour la chaîne B de l'insuline, ayant la structure indiqueée dans la revendication 17.

24. Méthode suivant l'une quelconque des revendications 19 à 23, caractérisée en ce que la séquence d'information d'ADN est inserée dans le vecteur de clonage réplicable en aval d'une séquence de promoteur de transcription d'ARNm fort.